# EUROPÄISCHE PATENTSCHRIFT

(11) **EP 1 310 270 B1**
(45) Veröffentlichungstag und Bekanntmachung des Hinweises auf die Patenterteilung: **29.03.2006**
(21) Anmeldenummer: 02023885.3
(22) Anmeldetag: 24.10.2002
(51) Int. Cl.: A61N 1/36

(54) **Implantierbares Muskelstimulationsgerät**
Implantable muscle stimulation apparatus
Appareil de stimulation musculaire implantable

(30) Priorität: 09.11.2001 DE 10155087
(43) Veröffentlichungstag der Anmeldung: 14.05.2003
(73) Patentinhaber: Pulsion Medical Systems AG, 81829 München (DE)
(72) Erfinder: Bumm, Rudolf, Dr., 81827 München (DE)
(74) Vertreter: Kehl, Günther

(56) Entgegenhaltungen:
- EP-A- 1 004 330
- WO-A-02/38217
- US-A- 5 188 104

## Beschreibung

Die vorliegende Erfindung bezieht sich auf ein implantierbares Muskelstimulationsgerät zur Behandlung der gastro-ösophagealen Refluxkrankheit (*gastro-esophageal reflux disease,* GERD).

Die Gastroösophageale Refluxkrankheit ist die häufigste Erkrankung des oberen Gastrointestinaltraktes.. Ursächlich besteht eine Insuffizienz des unteren Ösophagussphinkters mit Zurücklaufen von Magensäure, in schweren Fällen auch Duodenalsekret mit Pankreas- und Gallenfermenten in die Speiseröhre. Außerdem ist der physiologische Winkel (HIS-Winkel) zwischen Speiseröhre und Mageneingang bei vielen Patienten abgeflacht, und in vielen Fällen besteht ein Zwerchfellbruch, der zur Verlagerung des Mageneingangs über das Zwerchfell führt und dadurch den Reflux noch fördert. Bei länger einwirkendem Reflux kommt es bei Patienten zum Ausbilden Refluxösophagitis bis hin zum Umwandeln des Plattenepithels im Ösophagus zu Zylinderepithel. In diesem sogenannten Barrett-Ösophagus, der allgemein als Spätfolge einer langjährigen Refluxkrankheit aufgefaßt wird, kommt es häufig zum Entstehen von Adenokarzinomen des distalen Ösophagus. Diese Tumorart gehört derzeit zu den in der Häufigkeit am schnellsten zunehmenden Tumorarten der westlichen Welt.

Zur Behandlung der gastroösophagealen Refluxkrankheit können die Magensäuresekretion hemmende Medikamente eingesetzt werden. Diese führen zwar meist zu einer Abheilung der Refluxösophagitis, in der Regel kommt es jedoch nach dem Absetzen der Medikamente zu einem Rezidiv, so daß eine lebenslange Einnahme erforderlich ist. Die Behandlung ist für den Refluxpatienten teuer und umständlich in der Handhabung. Auch verhindert das Abblocken der Magensäure nicht den Rückfluß von Gallen- und Pankreasfermenten in den Ösophagus. Eine definitive medikamentöse Therapie, die nicht als Dauerbehandlung durchgeführt werden müßte, existiert nicht.

Die gängige chirurgische Therapie der Refluxkrankheit besteht im Herumlegen und Fixieren eines Magenanteils, des sogenannten Magenfundus, als Manschette um den Endteil des Ösophagus im Bereich des unteren Ösophagussphinkters. Als Folge dieses als Fundoplikatio bezeichneten Eingriffs ist bei vielen Patienten die Unfähigkeit des Aufstoßens und des Erbrechens festzustellen. Gelegentlich treten vermehrte Blähungen auf. Im Falle einer zu eng angelegten Manschette kommt es häufig zu Schluck- und Passagestörungen, die gelegentlich sogar einen erneuten operativen Eingriff nach sich ziehen. Bei zu weit angelegter Manschette kommt es zu einem Wiederauftreten von gastro-ösophagealem Reflux, und es entsteht ein Refluxrezidiv. Auch bei später Aufweitung der Manschette kommt es zu einem Rezidiv, das medikamentös oder durch Reoperation behandelt werden kann. Im Falle der Dislokation der Manschette wird der Antirefluxmechanismus normalerweise hyperkompetent, und die entstehenden Schluckstörungen und Schmerzen machen häufig eine Reoperation unumgänglich.

Aus US 5,716,385 ist ein implantierbarer Zwerchfellschrittmacher sowie dessen Verwendung zur Behandlung der Gastroösophagealen Refluxkrankheit bekannt. Der Zwerchfellschrittmacher reagiert auf elektromyographisch detektierte vorübergehende Relaxationen des unteren ösophagealen Sphinkters mit Stimulation der Muskeln des cruralen Zwerchfells, wodurch der Druck im Bereich des unteren Ösophagus erhöht wird. Mitunter wird jedoch der gastroösophageale Reflux nur vermindert, nicht jedoch vermieden.

In US 6,097,984 ist ein implantierbares Muskelstimulationsgerät der eingangs genannten Art offenbart, bei welchem der untere ösophageale Sphinktermuskel (*lower esophageal sphincter*, LES) in Abhängigkeit der Motilität des Ösophagus stimuliert wird. Das Muskelstimulationsgerät weist dabei eine Regelungscharakterstik auf, nach welcher eine hohe gemessene ösophageale Motilität die Stimulation des unteren ösophagealen Sphinkters vermindert, wodurch der Schließdruck sinkt und die Nahrungsaufnahme erleichtert wird. Eine niedrige gemessene ösophageale Motilität verstärkt die Stimulation des unteren ösophagealen Sphinkters, wodurch der Schließdruck steigt.

Der bekannte Regelungsansatz bringt jedoch einige größere Probleme mit sich. Zum einen können Schluckbewegungen mit einhergehender erhöhter Ösophagus-Motilität auch ohne Aufnahme von Nahrung erfolgen. Entsprechend kann durch Schlucken eine temporäre Lockerung des Sphinkters ausgelöst werden mit der Folge, daß durch unterbewußtes Schlucken im Schlaf der von Reflux-Patienten besonders gefürchtete nächtliche Reflux ausgelöst werden kann. Bereits Kaugummikauen nach einer Mahlzeit könnte eine ösophagus-motilitätsgeregelte Sphinkter-Stimulierung stark beeinträchtigen. Zum anderen ist bei Refluxpatienten die Ösophagusmotilität ohnehin häufig gestört. Die gestörte Ösophagusmotilität kann aufgrund der grundsätzlichen Regelungscharakterisitik dann zu einem dauerhaft hohen Schließdruck des unteren Ösophagus-Sphinkters und zu Dysphagie führen.

Ferner ist die Messung der Ösophagus-Motilität mittels implantierbarer Elektroden technisch schwierig umsetzbar. Insbesondere kann von einer detektierten Ösophagus-Welle kaum oder kaum zuverlässig geschlossen werden, ob überhaupt ein Bolus enthalten ist, oder nur Speichel geschluckt wurde, was zu der obengeschilderten Problematik führt.

Besonders schwerwiegend ist, daß ein Zweihöhleneingriff zur Installation der aus US 6,097,984 bekannten Vorrichtung erforderlich ist. Neben der Schaffung eines Zugangs zum unteren ösophagealen Sphinkter für die Implantation des Schrittmachers muß auch noch ein Zugang zur Anbringung der Ösphagussensoren erfolgen. Dies kann eine zusätzliche Lungenspiegelung oder eine Eröffnung des Zwerchfells sein, wobei insbesondere letzteres bei Refluxpatienten das Risiko zusätzlicher Komplikationen birgt. Generell kann die Anbringung von Sensoren am Ösophagus als nicht ungefährlich eingestuft werden.

Die Aufgabe der vorliegenden Erfindung besteht darin, eine Vorrichtung zu schaffen, die geeignet ist, gastroösophagealem Reflux entgegenzuwirken, und deren Einsatz die Anwendung herkömmlicher medikamentöser oder chirurgischer Therapien mit den oben angeführten Problemen vermeidbar macht.

Nach einem Aspekt der Erfindung weist ein implantierbares Muskelstimulationsgerät mindestens zwei implantierbare, zur Stimulation eines Sphinkters oder Sphinkterersatzes geeignete Stimulationselektroden, mindestens einen implantierbaren Sensor zur Ausgabe eines vom Füllungszustand des Magens abhängigen Sensorsignals, und ein Steuergerät, welches einen Impulsgeber zum Aufsteuern elektrischer Impulse auf die Stimulationselektroden sowie einen Signaleingang zum Empfang des Sensorsignals aufweist, auf. Dabei sind die Frequenz und/oder die Intensität der elektrischen Impulse entsprechend Anspruch 1 egelbar. Bei hohem Füllungszustand des Magens und entsprechend höherer Refluxgefahr bewirkt das Gerät somit einen höheren auf den Ösophagus ausgeübten Druck des Sphinkters oder Sphinkterersatzes, wohingegen bei leerem Magen die Nahrungsaufnahme durch Senken des Drucks erleichtert wird. Die Gefahr eines dauerhaft hohen Schließdrucks des unteren Ösophagus-Sphinkters besteht im Gegensatz zum Stand der Technik nicht. Ebenso bleiben bei der erfindungsgemäßen Abhängigkeit der Sphinkter-Stimulierung vom Magenfüllungszustand unerwünschte Auswirkungen von ohne Nahrungsaufnahme ausgeführtem Schlucken aus.

Gegenüber dem Stand der Technik besteht bei dem erfindungsgemäßen Muskelstimulationsgerät zudem der Vorteil, daß zu dessen Installation kein Zweihöhleneingriff erforderlich ist. Die Anbringung der Sensorik an der Magenwand und der Stimulationselektroden am unteren ösophagealen Sphinkter kann durch ein und dieselbe chirurgisch geschaffene Öffnung im Rahmen einer Bauchspiegelung erfolgen.

Nach einer vorteilhaften Weiterbildung der vorliegenden Erfindung handelt es sich bei dem Sensor um einen Dehnungsrezeptor, der vorzugsweise mindestens einen Dehnungsmeßstreifen aufweist.

Nach besonders bevorzugten, hierzu alternativen Weiterbildung der vorliegenden Erfindung weist der Sensor mindestens zwei, vorzugsweise drei bis fünf Meßelektroden zur Messung der Impedanz von zum Magen gehörigem Gewebe auf.

Vorzugsweise weist das Steuergerät mindestens eine Batterie auf. Zweckmäßig sind hier unter dem Gesichtspunkt der Langlebigkeit und der speicherbaren Energiedichte beispielsweise Lithium-Zellen.

Nach einer vorteilhaften Ausführungsform ist das Steuergerät mit mindestens einem integrierten Schaltkreis und/oder Mikroprozessor ausgestattet. Dies erlaubt einen hohen Grad an Miniaturisierung und erhöht so den Komfort für den Patienten.

In einer bevorzugten Weiterbildung weist das Muskelstimulationsgerät drei bis fünf Stimulationselektroden auf.

Besonders vorteilhaft ist es, das Steuergerät zusätzlich mit einen Steuereingang auszustatten, über welchen die Frequenz und/oder die Intensität der elektrischen Impulse durch ein externes Signal steuerbar sind. Beispielsweise kann der Patient so befähigt werden, in bestimmten Situationen, beispielsweise nach dem Einnehmen opulenter Mahlzeiten, das Gerät durch eine einfache Maßnahme auf maximale Sphinkterstimulation zu stellen. Auch eine Kalibrierung kann über den Steuereingang vorgenommen werden.

Ferner wird ein beschrieben, weiches Computerprogramm beschrieben, welches ausführbare Instruktionen zur Regelung der Frequenz und/oder Intensität von elektrischen Impulsen zur Stimulation eines Sphinkters oder Sphinkterersatzes in Abhängigkeit eines den Füllungszustand des Magens anzeigenden Sensorsignals eines implantierten Sensors aufweist. Dabei weist das Computerprogramm vorzugsweise eine Regelcharakteristik auf, wonach ein höherer Füllungszustand des Magens eine stärkere Stimulation des Sphinkters oder Sphinkterersatzes und somit einen höheren Schließdruck bewirkt.

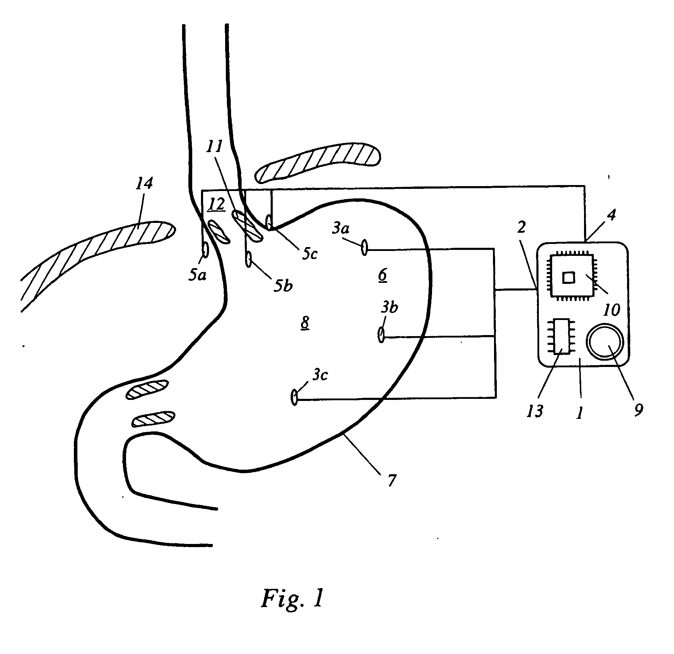

Nachstehend wird ein Ausführungsbeispiel der vorliegenden Erfindung anhand der schematischen, nicht maßstäblichen Zeichnung erläutert. Hierbei zeigt Fig. 1 den prinzipiellen Aufbau eines erfindungsgemäßen Muskelstimulationsgeräts im implantierten Zustand.

Das in Fig. 1 schematisch dargestellte Muskelstimulationsgerät besitzt ein Steuergerät 1 mit einem Eingang 2, an welchem die Sensorelektroden 3a, 3b, 3c angeschlossen sind, sowie einem Ausgang 4, welcher mit den Stimulationselektroden 5a, 5b, 5c verbunden ist. Mittels der Sensorelektroden 3a, 3b, 3c, welche im Bereich der großen Magenkurvatur 6 implantiert sind, kann fortlaufend die Impedanz der Magenwand 7 gemessen werden, über welchletztere das Steuergerät 1 den Füllungsszustand des Magens 8 ermittelt.

Das Steuergerät 1 weist eine austauschbare Lithiumbatterie 9 als elektrische Spannungsquelle sowie einen Mikroprozessor 10 und integrierte Schaltkreise (nicht dargestellt) auf und legt in Abhängigkeit des Füllungsszustand des Magens 8 Frequenz und/oder die Intensität elektrischer Impulse fest, welche zur direkten Stimulation des Sphinkters 11 (unteren Speiseröhrensphinkter) mittels der radial um den Sphinkter 11 plazierten Stimutationselektroden 5a, 5b, 5c dienen.

Für die Ausführung dieser Regelung ist in dem Steuergerät 1 ein mittels des Mikroprozessors 10 ausführbares Computerprogramm implementiert, welches eine Regelcharakteristik aufweist, wonach ein höherer Füllungszustand des Magens 8 eine stärkere Stimulation des Sphinkters 11 oder Sphinkterersatzes bewirkt. Der Sphinkter 11 reagiert auf die Stimulation durch Ausübung eines entsprechenden Drucks auf den Bereich des unteren Ösophagus 12. Wenn die Refluxgefahr aufgrund reichlicher vorausgegangener Nahrungsaufnahme des Patienten erhöht ist, wird so durch eine mittels entsprechender Stimualtion des Sphinkters 11 bewirkten Anhebung des Schließdrucks am unteren Ösophagus 12 der Gefahr entgegengewirkt. Bei nur wenig gefülltem Magen 8 erleichtert ein entsprechend verminderter Schließdruck die Nahrungsaufnahme. Das Computerprogramm kann beispielsweise auf einem nichtflüchtigen Speicherchip (EPROM) 13 in dem Steuergerät 1 gespeichert sein.

Das Steuergerät 1 ist über einen externen drahtlosen Signaleingang (nicht dargestellt) von außen kalibrierbar, so daß beispielsweise geeignete physiologische Obergrenzen für den maximal zu liefernden Stimulationsstrom festgelegt werden können. Hierfür weist das Computerprogramm eine entsprechend Kalibrierroutine auf. Darüberhinaus läßt sich über den externen drahtlosen Signaleingang die Stimulation des Sphinkters 11 und somit der Schließdruck am unteren Ösophagus 12 manuell auf den maximalen Wert einstellen.

Die Stimulationselektroden 5a, 5b, 5c wie auch die Sensorelektroden 3a, 3b, 3c können beispielsweise mittels einer Bauchspiegelung implantiert werden. Das Steuergerät 1 wird idealerweise wie ein herkömmlicher Herzschrittmacher in eine Gewebetasche unter dem Rippenbogen eingesetzt.

Bei unzureichender Stimulierbarkeit des Sphinkters 12 kann durch Präparation eines gestielten Muskellappens aus Eigengewebe, z.B. Muskulatur des Zwerchfells 14 des Patienten eine Sphinkterersatzmanschette hergestellt werden.

## Patentansprüche (Patentansprüche für folgende(n) Vertragsstaat(en): IT, ES)

1. Implantierbares Muskelstimulationsgerät zur Behandlung der gastro-ösophagealen Refluxkrankheit, aufweisend
(a) mindestens zwei implantierbare, zur Stimulation eines Sphinkters oder Sphinkterersatzes geeignete Stimulationselektroden (5a, 5b, 5c),
(b) mindestens einen implantierbaren Sensor zur Ausgabe eines Sensorsignals, und
(c) ein Steuergerät (1), welches einen Impulsgeber zum Aufsteuern elektrischer Impulse auf die Stimulationselektroden sowie einen Signaleingang (2) zum Empfang des Sensorsignals aufweist, wobei die Frequenz und/oder die Intensität der elektrischen Impulse in Abhängigkeit des Sensorsignals regelbar sind, **dadurch gekennzeichnet, dass** das Sensorsignal abhängig ist vom Füllungszustand des Magens.

2. Implantierbares Muskelstimulationsgerät nach Anspruch 1, **dadurch gekennzeichnet, daß** die Frequenz und/oder die Amplitude der elektrischen Impulse in Abhängigkeit des Sensorsignals so regelbar sind, daß ein höherer Füllungszustand des Magens (8) eine stärkere Stimulation des Sphinkters (11) oder Sphinkterersatzes und somit einen höheren Schließdruck bewirkt.

3. Implantierbares Muskelstimulationsgerät nach einem der vorangehenden Ansprüche, **dadurch gekennzeichnet, daß** es sich bei dem Sensor um einen Dehnungsrezeptor handelt.

4. Implantierbares Muskelstimulationsgerät nach Anspruch 3, **dadurch gekennzeichnet, daß** der Dehnungsrezeptor mindestens einen Dehnungsmeßstreifen aufweist.

5. lmplantierbares Muskelstimulationsgerät nach Anspruch 1, **dadurch gekennzeichnet, daß** der Sensor mindestens zwei Meßelektroden (3a, 3b, 3c) zur Messung der lmpedanz zum Magen (8) gehörigen Gewebes aufweist.

6. lmplantierbares Muskelstimulationsgerät nach Anspruch 5, **dadurch gekennzeichnet, daß** der Sensor 3 bis 5 Meßelektroden (3a, 3b, 3c) zur Messung der Impedanz der Magenwand (7) aufweist.

7. lmplantierbares Muskelstimulationsgerät nach einem der vorangehenden Ansprüche, **dadurch gekennzeichnet, daß** das Steuergerät (1) mindestens eine Batterie (9) aufweist.

8. Implantierbares Muskelstimulationsgerät nach einem der vorangehenden Ansprüche, **dadurch gekennzeichnet, daß** das Steuergerät (1) mindestens einen integrierten Schaltkreis aufweist.

9. lmplantierbares Muskelstimulationsgerät nach einem der vorangehenden Ansprüche, **dadurch gekennzeichnet, daß** das Steuergerät (1) mindestens einen Mikroprozessor (10) aufweist.

10. Implantierbares Muskelstimulationsgerät nach einem der vorangehenden Ansprüche, **dadurch gekennzeichnet, daß** das Muskelstimulationsgerät 3 bis 5 Stimulationselektroden (5a, 5b, 5c) aufweist.

11. Implantierbares Muskelstimulationsgerät nach einem der vorangehenden Ansprüche, **dadurch gekennzeichnet, daß** das Steuergerät (1) zusätzlich einen Steuereingang aufweist, über welchen die Frequenz und/oder die Intensität der elektrischen Impulse durch ein externes Signal steuerbar und/oder kalibrierbar sind.

## Patentansprüche (Patentansprüche für folgende(n) Vertragsstaat(en): DE, FR, GB)

1. Implantierbares Muskelstimulationsgerät zur Behandlung der gastro-ösophagealen Refluxkrankheit, aufweisend
(a) mindestens zwei implantierbare, zur Stimulation eines Sphinkters oder Sphinkterersatzes geeignete Stimulationselektroden (5a, 5b, 5c),
(b) mindestens einen implantierbaren Sensor zur Ausgabe eines vom Füllungszustand des Magens (8) abhängigen Sensorsignals, und
(c) ein Steuergerät (1), welches einen Impulsgeber zum Aufsteuern elektrischer Impulse auf die Stimulationselektroden sowie einen Signaleingang (2) zum Empfang des Sensorsignals aufweist,
**dadurch gekennzeichnet, daß** die Frequenz und/oder die Amplitude der elektrischen Impulse in Abhängigkeit des Sensorsignals so regelbar sind, daß ein höherer Füllungszustand des Magens (8) eine stärkere Stimulation des Sphinkters (11) oder Sphinkterersatzes und somit einen höheren Schließdruck bewirkt.

2. Implantierbares Muskelstimulationsgerät nach einem der vorangehenden Ansprüche, **dadurch gekennzeichnet, daß** es sich bei dem Sensor um einen Dehnungsrezeptor handelt.

3. Implantierbares Muskelstimulationsgerät nach Anspruch 2, **dadurch gekennzeichnet, daß** der Dehnungsrezeptor mindestens einen Dehnungsmeßstreifen aufweist.

4. Implantierbares Muskelstimulationsgerät nach Anspruch 1, **dadurch gekennzeichnet, daß** der Sensor mindestens zwei Meßelektroden (3a, 3b, 3c) zur Messung der Impedanz zum Magen (8) gehörigen Gewebes aufweist.

5. Implantierbares Muskelstimulationsgerät nach Anspruch 4, **dadurch gekennzeichnet, daß** der Sensor 3 bis 5 Meßelektroden (3a, 3b, 3c) zur Messung der Impedanz der Magenwand (7) aufweist.

6. Implantierbares Muskelstimulationsgerät nach einem der vorangehenden Ansprüche, **dadurch gekennzeichnet, daß** das Steuergerät (1) mindestens eine Batterie (9) aufweist.

7. Implantierbares Muskelstimulationsgerät nach einem der vorangehenden Ansprüche, **dadurch gekennzeichnet, daß** das Steuergerät (1) mindestens einen integrierten Schaltkreis aufweist.

8. lmplantierbares Muskelstimulationsgerät nach einem der vorangehenden Ansprüche, **dadurch gekennzeichnet, daß** das Steuergerät (1) mindestens einen Mikroprozessor (10) aufweist.

9. Implantierbares Muskelstimulationsgerät nach einem der vorangehenden Ansprüche, **dadurch gekennzeichnet, daß** das Muskelstimulationsgerät 3 bis 5 Stimulationselektroden (5a, 5b, 5c) aufweist.

10. Implantierbares Muskelstimulationsgerät nach einem der vorangehenden Ansprüche, **dadurch gekennzeichnet, daß** das Steuergerät (1) zusätzlich einen Steuereingang aufweist, über welchen die Frequenz und/oder die Intensität der elektrischen Impulse durch ein externes Signal steuerbar und/oder kalibrierbar sind.

## Claims (Claims for the following Contracting State(s): IT, ES)

1. Implantable muscle stimulation device for treating gastro-oesophageal reflux disease, having
(a) at least two implantable stimulation electrodes (5a, 5b, 5c) suitable for stimulating a sphincter or sphincter substitute,
(b) at least one implantable sensor for emitting a sensor signal, and
(c) a control device (1), having a pulse transmitter for supplying electrical pulses to the stimulation electrodes and a signal input (2) for receiving the sensor signal, wherein the frequency an/or the intensity of the electric pulses can be regulated as a function of the sensor signal, **characterised in that** the sensor signal depends on the fill state of the stomach,

2. Implantable muscle stimulation device according to claim 1, **characterised in that** the frequency and/or the amplitude of the electrical pulses can be regulated as a function of the sensor signal in such a way that a higher fill state of the stomach (8) causes a stronger stimulation of the sphincter (11) or sphincter substitute and therefore a higher closing pressure.

3. Implantable muscle stimulation device according to any one of the preceding claims, **characterised in that** the sensor is a dilation receptor.

4. Implantable muscle stimulation device according to claim 3, **characterised in that** the dilation receptor has at least one strain gauge.

5. Implantable muscle stimulation device according to claim 1, **characterised in that** the sensor has at least two measuring electrodes (3a, 3b, 3c) for measuring the impedance of tissue pertaining to the stomach (8).

6. Implantable muscle stimulation device according to claim 5, **characterised in that** the sensor has 3 to 5 measuring electrodes (3a, 3b, 3c) for measuring the impedance of the stomach wall (7).

7. Implantable muscle stimulation device according to any one of the preceding claims, **characterised in that** the control device (1) has at least one battery (9).

8. Implantable muscle stimulation device according to any one of the preceding claims, **characterised in that** the control device (1) has at least one integrated switching circuit.

9. Implantable muscle stimulation device according to any one of the preceding claims, **characterised in that** the control device (1) has at least one microprocessor (10).

10. Implantable muscle stimulation device according to any one of the preceding claims, **characterised in that** the muscle stimulation device has 3 to 5 stimulation electrodes (5a, 5b, 5c).

11. Implantable muscle stimulation device according to any one of the preceding claims, **characterised in that** the control device (1) also has a control input, via which the frequency and/or the intensity of the electrical pulses can be controlled and/or calibrated by an external signal.

## Claims (Claims for the following Contracting State(s): DE, FR, GB)

1. Implantable muscle stimulation device for treating gastro-oesophageal reflux disease, having
(a) at least two implantable stimulation electrodes (5a, 5b, 5c) suitable for stimulating a sphincter or sphincter substitute,
(b) at least one implantable sensor for emitting a sensor signal which depends on the fill state of the stomach (8), and
(c) a control device (1), having a pulse transmitter for supplying electrical pulses to the stimulation electrodes and a signal input (2) for receiving the sensor signal,
**characterised in that** the frequency and/or the amplitude of the electrical pulses can be regulated as a function of the sensor signal in such a way that a higher fill state of the stomach (8) causes a stronger stimulation of the sphincter (11) or sphincter substitute and therefore a higher closing pressure.

2. Implantable muscle stimulation device according to any one of the preceding claims, **characterised in that** the sensor is a dilation receptor.

3. Implantable muscle stimulation device according to claim 2, **characterised in that** the dilation receptor has at least one strain gauge.

4. Implantable muscle stimulation device according to claim 1, **characterised in that** the sensor has at least two measuring electrodes (3a, 3b, 3c) for measuring the impedance of tissue pertaining to the stomach (8).

5. Implantable muscle stimulation device according to claim 4, **characterised in that** the sensor has 3 to 5 measuring electrodes (3a, 3b, 3c) for measuring the impedance of the stomach wall (7).

6. Implantable muscle stimulation device according to any one of the preceding claims, **characterised in that** the control device (1) has at least one battery (9).

7. Implantable muscle stimulation device according to any one of the preceding claims, **characterised in that** the control device (1) has at least one integrated switching circuit.

8. Implantable muscle stimulation device according to any one of the preceding claims, **characterised in that** the control device (1) has at least one microprocessor (10).

9. Implantable muscle stimulation device according to any one of the preceding claims, **characterised in that** the muscle stimulation device has 3 to 5 stimulation electrodes (5a, 5b, 5c).

10. Implantable muscle stimulation device according to any one of the preceding claims, **characterised in that** the control device (1) also has a control input, via which the frequency and/or the intensity of the electrical pulses can be controlled and/or calibrated by an external signal.

## Revendications (Revendications pour l'(les) Etat(s) contractant(s) suivant(s): IT, ES)

1. Appareil de stimulation musculaire implantable pour le traitement de la maladie du reflux gastro-oesophagien, présentant :
(a) au moins deux électrodes de stimulation implantables (5a, 5b, 5c), appropriées pour la stimulation d'un sphincter ou d'un sphincter de remplacement ;
(b) au moins un détecteur implantable pour l'émission d'un signal de détecteur ; et
(c) un appareil de commande (1), lequel présente un générateur d'impulsions pour la génération d'impulsions électriques sur les électrodes de stimulation ainsi qu'une arrivée de signal (2) pour la réception du signal de détecteur, la séquence et/ou l'intensité des impulsions électriques étant réglables en fonction du signal de détecteur,
**caractérisé par le fait que** le signal de détecteur est dépendant de l'état de remplissage de l'estomac.

2. Appareil de stimulation musculaire implantable selon la revendication 1, **caractérisé par le fait que** la fréquence et/ou l'amplitude des impulsions électriques sont réglables en fonction du signal de détecteur de telle sorte qu'un état de remplissage plus élevé de l'estomac (8) provoque une stimulation plus forte du sphincter (11) ou du sphincter de remplacement et, par conséquent, une pression de fermeture plus élevée.

3. Appareil de stimulation musculaire implantable selon l'une des revendications précédentes, **caractérisé par le fait qu'**il s'agit, dans le cas du détecteur, d'un récepteur d'allongement.

4. Appareil de stimulation musculaire implantable selon la revendication 3, **caractérisé par le fait que** le récepteur d'allongement présente au moins une jauge d'allongement.

5. Appareil de stimulation musculaire implantable selon la revendication 1, **caractérisé par le fait que** le détecteur présente au moins deux électrodes de mesure (3a, 3b, 3c) pour la mesure de l'impédance du tissu appartenant à l'estomac (8).

6. Appareil de stimulation musculaire implantable selon la revendication 5, **caractérisé par le fait que** le détecteur présente 3 à 5 électrodes de mesure (3a, 3b, 3c) pour la mesure de l'impédance de la paroi d'estomac (7) .

7. Appareil de stimulation musculaire implantable selon l'une des revendications précédentes, **caractérisé par le fait que** l'appareil de commande (1) présente au moins une batterie (9).

8. Appareil de stimulation musculaire implantable selon l'une des revendications précédentes, **caractérisé par le fait que** l'appareil de commande (1) présente au moins un circuit de commutation intégré.

9. Appareil de stimulation musculaire implantable selon l'une des revendications précédentes, **caractérisé par le fait que** l'appareil de commande (1) présente au moins un microprocesseur (10).

10. Appareil de stimulation musculaire implantable selon l'une des revendications précédentes, **caractérisé par le fait que** l'appareil de stimulation musculaire présente 3 à 5 électrodes de stimulation (5a, 5b, 5c).

11. Appareil de stimulation musculaire implantable selon l'une des revendications précédentes, **caractérisé par le fait que** l'appareil de commande (1) présente en outre une entrée de commande, par l'intermédiaire de laquelle la fréquence et/ou l'intensité des impulsions électriques sont commandables et/ou calibrables par un signal externe.

## Revendications (Revendications pour l'(les) Etat(s) contractant(s) suivant(s): DE, FR, GB)

1. Appareil de stimulation musculaire implantable pour le traitement de la maladie du reflux gastro-oesophagien, présentant :
(a) au moins deux électrodes de stimulation implantables (5a, 5b, 5c), appropriées pour la stimulation d'un sphincter ou d'un sphincter de remplacement ;
(b) au moins un détecteur implantable pour l'émission d'un signal de détecteur dépendant de l'état de remplissage de l'estomac (8) ; et
(c) un appareil de commande (1), lequel présente un générateur d'impulsions pour la génération d'impulsions électriques sur les électrodes de stimulation ainsi qu'une arrivée de signal (2) pour la réception du signal de détecteur,
**caractérisé par le fait que** la fréquence et/ou l'amplitude des impulsions électriques sont réglables en fonction du signal du détecteur de telle sorte qu'un état de remplissage plus élevé de l'estomac (8) provoque une stimulation plus forte du sphincter (11) ou du sphincter de remplacement et, par conséquent, une pression de fermeture plus élevée.

2. Appareil de stimulation musculaire implantable selon l'une des revendications précédentes, **caractérisé par le fait qu'**il s'agit, dans le cas du détecteur, d'un récepteur d'allongement.

3. Appareil de stimulation musculaire implantable selon la revendication 2, **caractérisé par le fait que** le récepteur d'allongement présente au moins une jauge d'allongement.

4. Appareil de stimulation musculaire implantable selon la revendication 1, **caractérisé par le fait que** le détecteur présente au moins deux électrodes de mesure (3a, 3b, 3c) pour la mesure de l'impédance du tissu appartenant à l'estomac (8).

5. Appareil de stimulation musculaire implantable selon la revendication 4, **caractérisé par le fait que** le détecteur présente 3 à 5 électrodes de mesure (3a, 3b, 3c) pour la mesure de l'impédance de la paroi d'estomac (7).

6. Appareil de stimulation musculaire implantable selon l'une des revendications précédentes, **caractérisé par le fait que** l'appareil de commande (1) présente au moins une batterie (9).

7. Appareil de stimulation musculaire implantable selon l'une des revendications précédentes, **caractérisé par le fait que** l'appareil de commande (1) présente au moins un circuit de commutation intégré.

8. Appareil de stimulation musculaire implantable selon l'une des revendications précédentes, **caractérisé par le fait que** l'appareil de commande (1) présente au moins un microprocesseur (10).

9. Appareil de stimulation musculaire implantable selon l'une des revendications précédentes, **caractérisé par le fait que** l'appareil de stimulation musculaire présente 3 à 5 électrodes de stimulation (5a, 5b, 5c).

10. Appareil de stimulation musculaire implantable selon l'une des revendications précédentes, **caractérisé par le fait que** l'appareil de commande (1) présente en outre une entrée de commande, par l'intermédiaire de laquelle la fréquence et/ou l'intensité des impulsions électriques sont gouvernables et/ou calibrables par un signal externe.
